# EUROPEAN PATENT APPLICATION

(11) **EP 0 919 192 A2**
(43) Date of publication of application: **02.06.1999**
(21) Application number: 98309550.6
(22) Date of filing: 23.11.1998
(51) Int. Cl.: A61B 10/00

(54) **Biopsy instrument including tip for tissue dilation**

(30) Priority: 24.11.1997 US 976807
(71) Applicant: ETHICON ENDO-SURGERY, INC., Cincinnati, OH 45242-2839 (US)
(72) Inventor: Erickson, Paul L., Cincinnati, Ohio 45236 (US); Houser, Kevin L., Centerville, Ohio 45469 (US)
(74) Representative: Mercer, Christopher Paul

(57) **Abstract**

A surgical instrument for excising a targeted tissue mass to be biopsied from adjacent bodily tissue of a surgical patient is disclosed. The instrument has an elongated shaft to which is attached a penetrating member having a penetrating tip (16) to facilitate the insertion of the shaft adjacent the targeted tissue mass. The instrument also includes a tissue receiving chamber (25) to receive the targeted tissue mass to be biopsied, and a tissue cutter (26) movable from first to second positions to excise the targeted tissue received in the tissue receiving chamber. The penetrating tip includes a blade (19) having first and second planar surfaces (20,21) generally parallel to each other. The blade reduces the forces necessary to penetrate during insertion of the instrument through the tissue layers for positioning adjacent the biopsy site. It also serves to facilitate the dilation of the tissue during insertion. The reduced force to penetrate coupled with tissue dilation reduce patient trauma and hasten recovery time for the patient.

## Description

### Background of the Invention

This invention relates to an instrument for facilitating the removal of a suspect lesion for a subsequent biopsy to determine malignancy. In particular, it relates to such an instrument which can excise the suspect lesion and remove it for subsequent biopsy in a minimally invasive manner. Specifically, this invention relates to such an instrument which can minimize the trauma associated with inserting the instrument to position it adjacent the suspect lesion.

Statistics currently reveal that one in nine American women will develop breast cancer. It is the leading cause of cancer deaths in women between the ages of 40-55, and the second leading cause of cancer deaths in women overall. Unfortunately, breast cancer will only be diagnosed in about one in eight women, and one in thirty will die of this disease. Breast cancer does occur in men but it is much more uncommon.

A biopsy of the breast is often indicated if suspicious tissue is detected. Biopsy requests stem from a screening process generally performed via a physical examination to detect a palpable lesion or a mammogram for the detection of a nonpalpable lesion. Five out of six biopsies performed return benign indications.

The goal of the biopsy is the discovery and accurate diagnosis of benignity or malignancy of suspect tissue. Survival rates are significantly greater with early detection. However, as detection is attempted in earlier stages of carcinoma development, accurate sampling of the lesion becomes more difficult due to small lesion size. Ideally, a sufficiently large sample size is taken to accurately diagnosis disease state with minimal trauma (physical deformity or scarring) to the patient.

The current standard for care is a significantly invasive procedure known as "Open Excisional Biopsy". Prior to an open excisional biopsy for a nonpalpable mass, an interventional radiologist will initially take mammograms to place a guidewire which directs the surgeon to the area of excision. Mammograms are then taken to assure that the guidewire is accurately placed in or near the desired biopsy site. The guidewire has a barbed end in order maintain its position at the desired site and is fed through an elongated needle.
The general surgeon applies local or, more commonly, general anesthesia, during an open excisional biopsy. Dissection is made through the skin and subcutaneous fat and the flap is undercut and retracted. Incision length, during an open excisional biopsy generally runs between 2-5 inches and is dependent on the location of the lesion, breast characteristics, and surgeon technique. The breast parenchyma is then dissected along the guidewire until the site of the specimen is exposed. The specimen, generally spherical in shape centered but the barbed end of the guidewire, and roughly 1 inch in diameter, is manually excised.

The specimen size taken during an open excisional biopsy is adequate, however deformation (scarring and subsequent shape of the breast) and potential "seeding" of the cancerous cells is often a large concern. Accuracy is generally acceptable, however the guidewire often moves from its original position during the excisional process leading to decreased accuracy and the need for larger specimens.

New technologies are being developed to assist the early detection of breast cancer. The technologies are primarily focused on imaging of nonpalpable tissue and accessing the tissue with minimally invasive techniques for biopsy. The cost and trauma associated with open excisional biopsy is minimized with these technologies.

The two primary imaging technologies for assisting minimally invasive breast biopsy of a nonpalpable mass are ultrasound and stereotactic X-rays. Ultrasound accurately guides a hand-held core biopsy needle (CBN) to the biopsy site. Stereotactic imaging is performed with the patient lying prone on a stereotactic table and X-rays are taken at 15 degrees off-axis on each side of the breast. The X-rays are fed into a digital signal imaging system to create a 3-D reference; the location for biopsy is triangulated and coordinates are fed to the stereotactic table.

The actual specimen removal in the minimally invasive approach is performed in any one of the following ways.

TruCut® Needle Method - The "TruCut" needle method utilizes a small diameter needle which is inserted to a desired depth, actuated, and removed. The product for practicing this method has two co-axial tubular members. The outer member is retracted exposing a lateral opening in the inner member. The elastic property of the tissue causes tissue to enter the hollow core of the inner member. The outer member is then advanced forward, shearing the tissue sample which has been trapped in the inner member. Inherent disadvantages to this technique are the need for multiple insertions with questionable accuracy and extremely limited tissue sample sizes. The TruCut® needle and its method of use are described in U.S. Patent 4,958,625.

Surface to Site Core Sampling (SSCS) - The SSCS method attempts to maximize accuracy through the increase in sample size while maintaining controlled guidance. A cylindrical cutting tube is advanced through an opening in the skin. As it moves forward, it creates a continuous cylindrical core sample until it reaches the depth desired by the surgeon. Upon completion of the linear motion, the distal end of the sample is transected, and the device is withdrawn with the sample within the cutting element. The critical disadvantage to this approach is the trauma and disfigurement associated with excision of the healthy tissue superior to the site of questionable tissue.

Percutaneous Core Biopsy (PCB) - The PCB method attempts to obtain the advantages of single insertion and large sample size of the SSCS approach, while minimizing disfigurement as achieved by the TruCut® needle method. As in the case of a TruCut® needle device, the PCB has multiple co-axial tubular members. However, one of the members remains stationary, eliminating the need for multiple insertions. Samples are obtained in a controlled contiguous manner which ultimately achieves the desire for obtaining large tissue samples at the questionable site.

A recent minimally invasive approach for excising a portion of a targeted lesion is described in U.S. Patent 5,526,822. The biopsy apparatus described in this patent has a outer biopsy cannula with a penetrating member for positioning adjacent the targeted lesion. The biopsy cannula has a lateral tissue receiving port, and a vacuum is drawn through the port for pulling tissue into the port. An inner cutting cannula moves forwardly to excise the tissue received in the port. With the aid of a vacuum drawn on the inner cutting cannula, the inner cutting cannula retains the excised tissue as the inner cannula is retracted for tissue removal, and the outer biopsy cannula remains stationary. Following removal, the outer biopsy cannula can be rotated relative to a fixed housing and multiple samples can therefore be taken circumferentially without the need for multiple insertions and withdrawals of the biopsy cannula. The biopsy apparatus and its method of use as described in the '822 patent are exemplified in the Mammotome® Breast Biopsy System.

Although minimally invasive approaches for taking biopsy samples are now available, instruments and their methods of use which can further reduce trauma to the patient are still desirable. In particular, such an instrument would represent an advance in the state of the art if it could reduce the trauma associated with inserting the instrument through the various tissue layers to position the distal end of the instrument adjacent the targeted lesion for biopsy.

### Summary of the Invention

The invention is a surgical instrument for excising a targeted tissue mass to be biopsied from adjacent bodily tissue of a surgical patient. The instrument comprises an elongated shaft, a penetrating member, a tissue receiving chamber and a tissue cutter.

The elongated shaft of the instrument has proximal and distal ends. When the instrument is positioned to excise the targeted tissue mass, the proximal end of the elongated shaft is positioned externally of the surgical patient and the distal end of the elongated shaft is positioned adjacent the targeted tissue mass.

The penetrating member is affixed to the distal end of the elongated shaft. The penetrating member has a penetrating tip at a distal end of the penetrating member to facilitate the insertion of the distal end of the elongated shaft adjacent the targeted tissue mass.

The tissue receiving chamber of the instrument is in communication with the distal end of the elongated shaft for receiving the targeted tissue mass to be biopsied or a portion of the mass.

The tissue cutter has a distal cutting edge surface. It is movable relative to the penetrating member from a first position where the cutting edge surface is disposed proximally of the tissue receiving chamber to a second position located distally of the first position where the cutting edge surface has excised the targeted tissue mass to be biopsied or the portion of the mass within the tissue receiving chamber from the adjacent bodily tissue.

Importantly, the penetrating tip includes a blade having first and second planar surfaces generally parallel to each other. In the most preferred embodiment, the penetrating tip of the penetrating member has an exterior conical or pyramidal surface, and the blade extends outwardly from this surface and generally parallel to the longitudinal axis of the elongated shaft.

The inclusion of the blade for the penetrating tip of the penetrating member of the instrument of this invention significantly reduces trauma to the patient during the biopsy procedure. This is so because the blade may reduce the penetration force necessary to insert the instrument through the various layers of tissue, and also enhances the ability of the tissue layers to dilate as the insertion of the instrument progresses toward the interior biopsy site. This reduced force to penetrate, coupled with the ability to more effectively dilate the tissue during penetration, reduces patient trauma and correspondingly speeds up patient recovery.

The surgical instrument of this invention can be used where it is necessary or desirable to excise a targeted lesion from adjacent bodily tissue during a surgical biopsy procedure. In particular, the instrument is especially advantageous where it is desired to perform a biopsy procedure in a minimally invasive manner and where trauma to the patient is sought to be minimized,

### Brief Description of the Drawings

Fig. 1 is an isometric view of the distal end of a preferred embodiment of the instrument of this invention.
Fig. 2 is a centerline section view of the instrument of Fig. 1 in the pre-operative position.
Fig. 3 is a centerline section view of the instrument after insertion into breast tissue. The distal end thereof is placed inside the targeted tissue mass.
Fig. 4 is a centerline section view of the instrument with the shaft distal end moved distally to open the tissue receiving chamber.
Fig. 5 is a centerline section view of the instrument with vacuum applied to the tissue receiving chamber to draw in and capture a sample.
Fig. 6 is a centerline section view of the instrument with the tissue cutter moved distally to separate a tissue sample from the breast.

### Detailed Description of the Preferred Embonidment

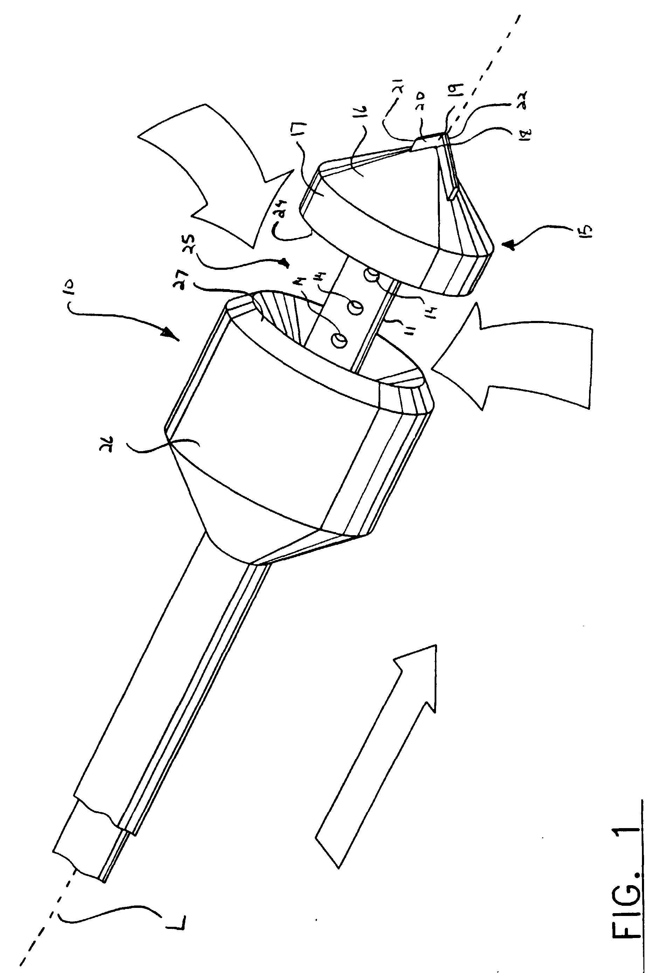

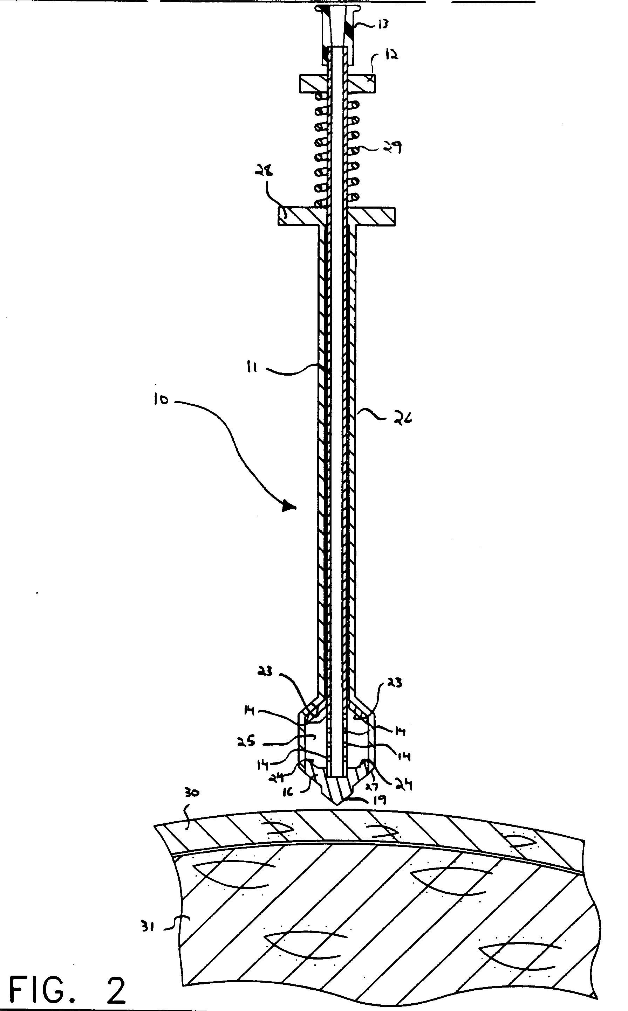

Referring initially to Figures 1 and 2, there is seen the preferred surgical instrument 10 of this invention for excising a targeted tissue mass to be biopsied from adjacent tissue of a patient. The distal end of the instrument is shown in Figure 1, and a sectional view of the instrument in close proximity to the patient is shown in Figure 2. The instrument has a long shaft 11 in the form of a hollow tube. At the proximal end of the shaft, there is mounted a shaft base 12 to grip the instrument, and a vacuum adapter 13 to connect the shaft to a vacuum source (see Figure 2 for a depiction of the shaft base and vacuum adapter; the vacuum source is not shown). At the distal end of the shaft, which can be observed in both Figures 1 and 2, there are a plurality of vacuum orifices 14 to pull a vacuum through the tube when the vacuum source is activated.

Still referring to Figures 1 and 2, affixed to the distal end of the shaft is a penetrating member 15 to facilitate the insertion of the instrument into tissue. The penetrating member has a penetrating tip 16 conducive to expanding an initial incisional opening made in the tissue to enable insertion of the instrument into tissue without the need to apply undo pressure. The penetrating tip 16 has a circular tip base 17 and an apex 18 spaced from the tip base. The penetrating tip converges toward the apex from the tip base in the distal direction. The penetrating tip is preferably conical in shape, and is ideally a right circular cone.

Importantly, a blade 19 extends outwardly from the penetrating tip. The blade reduces the forces necessary to insert the instrument into and through the tissue, therefore providing the user with greater control and an enhanced sense of security during insertion. The blade is of the razor blade type, having first and second planar surfaces, 20 and 21, respectively, generally parallel to each other. The first and second surfaces converge to a linear cutting edge 22. The blade is generally parallel to the longitudinal axis of the shaft (the shaft longitudinal axis is depicted in Figure 1 as "L"). The blade preferably intersects the apex of the penetrating tip. The penetrating tip and the blade may be made of a unitary, one-piece construction using conventional molding techniques. Alternatively, the blade may be made separately from the penetrating tip and affixed to the penetrating tip using conventional techniques such as insert molding.

Continuing to focus on Figures 1 and 2, extending outwardly from the shaft 11 adjacent the proximal-most vacuum orifice is a tissue stop 23. The underside surface 24 of the penetrating member also acts as a tissue stop. The two stops face each other and define a tissue receiving chamber 25 between the stops for receiving all or a portion of the targeted tissue mass to be biopsied.

A tissue cutter 26 is concentrically mounted about the shaft 11 for sliding movement between a first proximal position to a second distal position. The cutter has a distal cutting edge surface 27 to excise tissue received in the tissue receiving chamber 25. In Figure 1, the cutter 26 is shown in its first retracted position, and the cutting edge surface 27 is located proximally of the tissue receiving chamber so that tissue can be received in the chamber. In Figure 2, the cutter is shown in its second distal position where the cutter fully encloses the tissue receiving chamber. The proximal and of the cutter has a cutter base 28 for gripping the instrument, and moving the cutter between its first and second positions. A cutter spring 29 is located between the shaft base 12 and the cutter base 28. Accordingly, the cutter 26 is biased distally toward its second position.

As illustrated in Figure 1, and in accordance with the directional arrows illustrated in Figure 1, the instrument is inserted in the distal direction where the blade and subsequently the penetrating tip provide the opening through which the instrument may be inserted. When vacuum is applied, tissue is attracted against the distal end of the shaft 11 at the vacuum orifics 14 within the tissue receiving chamber 25

As illustrated in Figure 2, when a biopsy is desired to be taken, the instrument, and specifically the blade 19 extending from the penetrating tip 16, is aligned with the desired puncture site. More specifically, when a breast biopsy is desired to be taken, the blade is positioned adjacent the outer skin layer 30 of the breast, for the ultimate insertion of the instrument into the percutaneous breast tissue 31 and the suspect lesion. Normally, a scalpel is used to make a skin incision prior to insertion of the biopsy instrument. The flat blade eliminates the need for a scalpel because the flat blade can be used to create the skin nick. The cutter of the instrument is allowed to remain in its normally biased, second distal position fully enclosing the tissue receiving chamber 25.

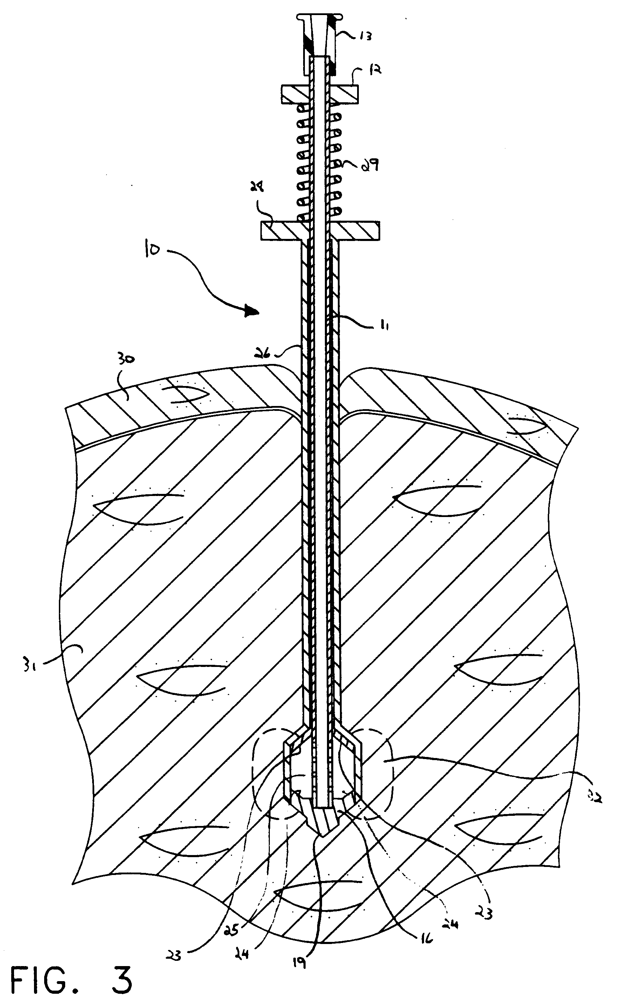

Referring now to Figure 3, the instrument 10 is inserted into the breast and the distal end of the instrument is positioned at the targeted tissue mass 32. In this position, the proximal end of the shaft is located externally of the patient so that the instrument can be manipulated and further positioned, and the distal end of the shaft is positioned adjacent the targeted tissue mass.

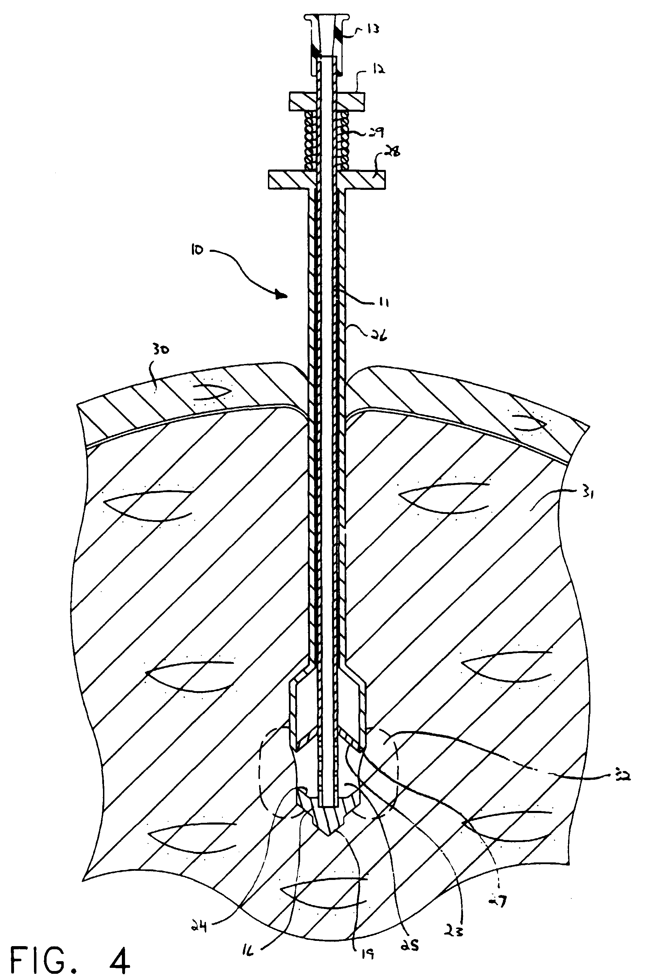

Turning to Figure 4, once the instrument is properly positioned within the breast, the cutter is retracted from its second extended position to its first proximal position. When the cutter is retracted, the tissue receiving chamber is uncovered. The cutter is retracted when a proximal force is applied to the cutter base 28 while holding the shaft base 12 stationary to compress the cutter spring.

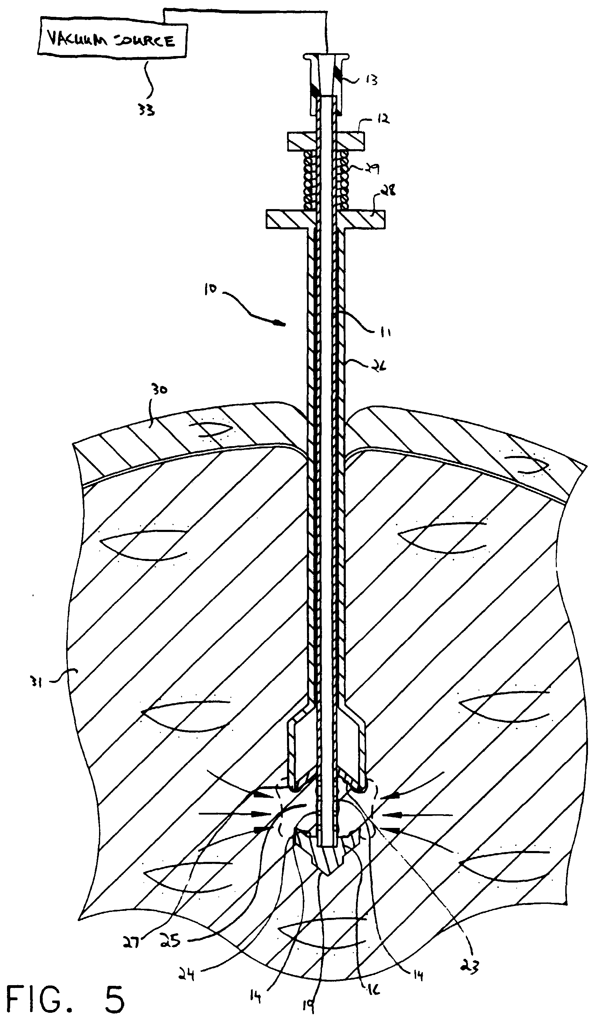

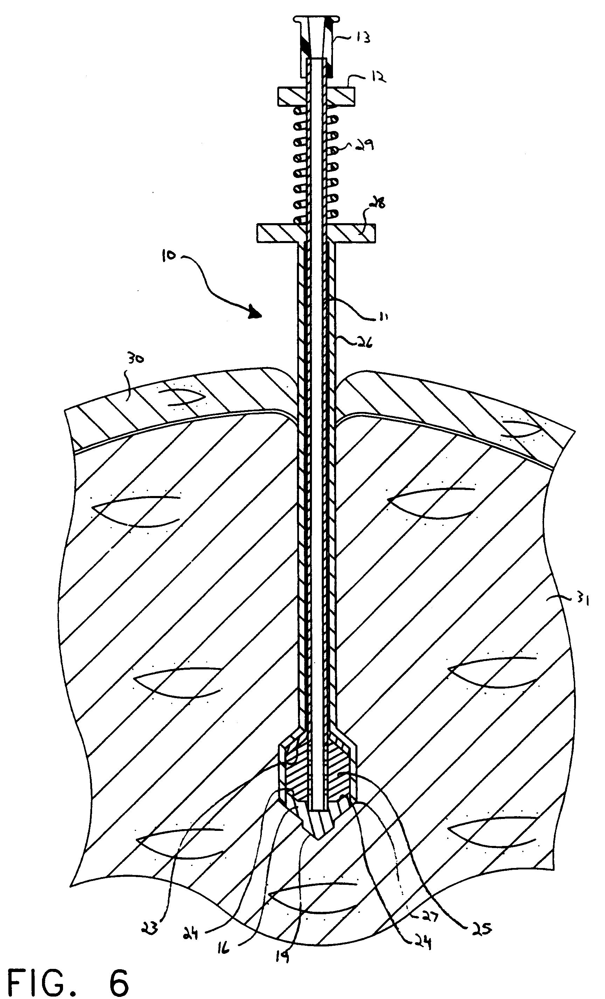

Next, as illustrated in Figure 5, the vacuum adapter 13 is attached to a vacuum source 33, and the vacuum source is activated while maintaining the cutter in its retracted first position. The vacuum draws the tissue into the tissue receiving chamber 25 as depicted by the directional arrows in Figure 5 to capture the tissue sample. Once the sample is captured, the proximal force applied to the cutter base 28 to compress the spring 29 is removed, releasing the cutter from its first position to its second extended position. As it moves distally, the distal cutting edge surface 27 of the cutter excises the tissue captured within the tissue receiving chamber of the instrument (see Figure 6). When the sample is fully excised, the instrument may be removed from the breast, and the excised tissue can subsequently be removed from the tissue receiving chamber 25.

Although this invention has been described in connection with its most preferred embodiment, numerous additional embodiments are within the scope and spirit of the invention in its broadest form. The true scope and content of the invention should not be construed to be limited by this preferred embodiment, but should be assessed in connection with the claims defining the invention which follow.

## Claims

1. A surgical instrument for excising a targeted tissue mass to be biopsied from adjacent bodily tissue of a surgical patient, said instrument comprising:
a) an elongated shaft having proximal and distal ends, and when said instrument is positioned to excise the targeted tissue mass, the proximal end of said elongated shaft is positioned externally of the surgical patient and the distal end of said elongated shaft is positioned adjacent the targeted tissue mass;
b) a penetrating member affixed to the distal end of said elongated shaft, said penetrating member having a penetrating tip at a distal end of said penetrating member to facilitate the insertion of the distal end of the said elongated shaft adjacent the targeted tissue mass;
c) a tissue receiving chamber in communication with the distal end of said elongated shaft for receiving the targeted tissue mass to be biopsied or a portion thereof; and
d) a tissue cutter having a distal cutting edge surface, said cutter movable relative to said penetrating member from a first position wherein said cutting edge surface is disposed proximally of said tissue receiving chamber to a second position located distally of said first position wherein said cutting edge surface has excised the targeted tissue mass to be biopsied or the portion thereof within said tissue receiving chamber from the adjacent bodily tissue;
wherein said penetrating tip includes a blade having first and second planar surfaces generally parallel to each other.

2. The instrument of Claim 1 wherein said elongated shaft has a shaft longitudinal axis, and said blade of said penetrating tip is generally parallel to the shaft longitudinal axis.

3. The instrument of Claim 2 wherein said penetrating tip further includes a tip base and an apex spaced from said tip base.

4. The instrument of Claim 3 wherein said tip base converges toward said apex in a distal direction.

5. The instrument of Claim 4 wherein said penetrating tip has an exterior conical surface.

6. The instrument of Claim 5 wherein said blade extends outwardly from said exterior surface.
